# EUROPEAN PATENT APPLICATION

(11) **EP 4 099 132 A1**
(43) Date of publication of application: **07.12.2022**
(21) Application number: 21193871.7
(22) Date of filing: 30.08.2021
(51) Int. Cl.: G06F 1/20, H05K 7/20, A61L 9/22

(54) **ELECTRONIC DEVICE**

(30) Priority: 01.06.2021 JP 2021092620
(71) Applicant: Dynabook Inc., Koto-ku Tokyo 135-8505 (JP)
(72) Inventor: CHEN, Peter, Tokyo, 135-8505 (JP); CHIU, Brad, Tokyo, 135-8505 (JP); LIN, Walter, Tokyo, 135-8505 (JP); LIN, Jonathan, Tokyo, 135-8505 (JP); UCHIYAMA, Isamu, Tokyo, 135-8505 (JP)
(74) Representative: Henkel & Partner mbB

(57) **Abstract**

According to one embodiment, an electronic device includes a housing (12) having a wall portion (18d) with a vent port (26), a plurality of electronic components disposed in the housing and including a heat-generating component (36), a cooling fan (FA1, FA2) including a discharge port opposing the vent port and an intake port opening into the housing and disposed in the housing to suction internal air of the housing and exhausting the air from the discharge port towards the vent port, and an ion generator (50) disposed in the housing and between the vent port and the discharge port of the cooling fan and including a passage channel communicating with the discharge port and the vent port and a discharge electrode exposed in the passage channel.

## Description

### FIELD

Embodiments of the invention generally relate to an electronic device.

### BACKGROUND

In recent years, a great number of air purification devices have been commercialized, some of which are desktop personal computers and notebook personal computers with air purification functions.

Desktop personal computers have a high degree of freedom in their internal space, and it is relatively easy to mount an ion generator in addition to the components that make up the computer system.

On the other hand, notebook personal computers have been downsized further, thus making it difficult to mount an ion generator therein. Under these circumstances, there has been proposed a technique which makes it possible to exclusively mount a module-type ion generator having the same shape as that of an optical disk drive (ODD) and the ODD.

The conventional technology requires a dedicated fan for ion diffusion, which means that space must be provided in the personal computer for the ion generator and the dedicated fan for ion diffusion, making it difficult to achieve both downsizing the product and securing space.

### BRIEF DESCRIPTION OF THE DRAWINGS

FIG. 1 is a perspective view showing a notebook-shaped personal computer according to a first embodiment.
FIG. 2 is a perspective view showing the inside of a housing of the personal computer.
FIG. 3 is a plan view schematically showing a cooling fan, an ion generator and a cooling mechanism of the personal computer.
FIG. 4 is a perspective view showing the cooling fan, the ion generator and an ion generation control circuit.
FIG. 5 is a cross sectional view of a portion of the ion generator taken along line A-A in FIG. 3.
FIG. 6 is a block diagram schematically showing a control system of the personal computer.
FIG. 7 is a plan view schematically showing a cooling fan, an ion generator and a cooling mechanism of the personal computer according to a second embodiment.
FIG. 8 is a perspective view showing a cooling fan, an ion generator and a cooling mechanism of a personal computer according to a third embodiment.
FIG. 9 is a plan view schematically showing a cooling fan, an ion generator and a cooling mechanism of a personal computer according to a third embodiment.
FIG. 10 is a perspective view schematically showing a cooling fan, an ion generator and a cooling mechanism of a personal computer according to a fourth embodiment.
FIG. 11 is perspective view showing an ion generator according to a modified example.

### DETAILED DESCRIPTION

Various embodiments will be described hereinafter with reference to the accompanying drawings. In general, according to one embodiment, an electronic device comprises a housing including a wall portion with a vent port; a plurality of electronic components including a heat-generating component, disposed within the housing; a cooling fan including a discharge port opposing the vent port and an intake port opening into the housing, and arranged inside the housing to suction internal air of the housing and discharge the air from the discharge port toward the vent port; and an ion generator disposed inside the housing between the discharge port of the cooling fan and the vent port, and including a passage channel communicating with the discharge port and the vent port and discharge electrodes exposed in the passage channel.

The disclosure is merely an example, and proper changes in keeping with the scope of the invention, which are easily conceivable by a person of ordinary skill in the art, come within the scope of the invention as a matter of course. In addition, in some cases, in order to make the description clearer, the widths, thicknesses, shapes and the like, of the respective parts are illustrated schematically in the drawings, rather than as an accurate representation of what is implemented. However, such schematic illustration is merely exemplary, and in no way restricts the interpretation of the invention. In addition, in the specification and drawings, the same elements as those described in connection with preceding drawings are denoted by like reference numbers, and detailed description thereof is omitted unless necessary.

### (First Embodiment)

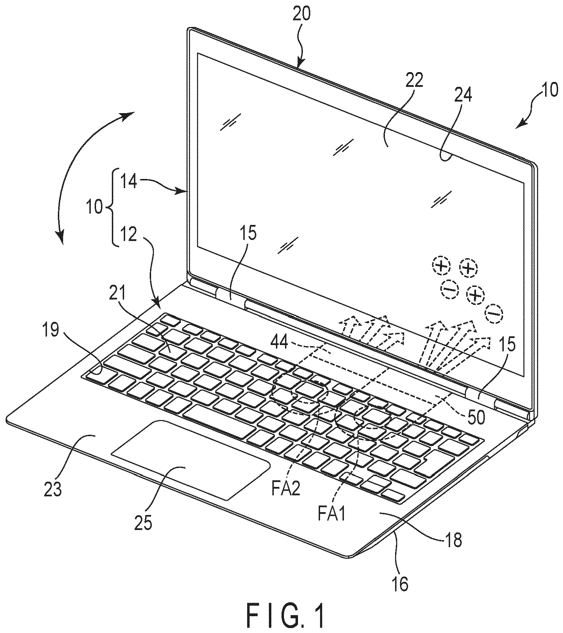

FIG. 1 is a perspective view showing a notebook-type personal computer (hereinafter referred to as a "PC") according to a first embodiment as an example of an electronic device.

As shown in FIG. 1, a PC 10 comprises a first housing 12 and a second housing 14. The first housing 12 and the second housing 14 are joined together so as to be pivotable with respect to each other by a pair of hinges 15. In this embodiment, the first and second housings 12 and 14 are both formed into a flat and substantially rectangular parallelepiped shape. The pair of hinges 15 are provided on long-side edges of respective housings, so as to be spaced apart in the longitudinal direction.

The first housing 12 comprises a base 16 with a rectangular bottom wall, and a top cover 18 fitted to the base 16. The top cover 18 includes a rectangular-shaped top wall and a plurality of side walls standing along the side edges of the top wall, integrated as one body. The first housing 12 is made of synthetic resin or metal.

A substantially rectangular opening 19 is formed in a central portion of the top cover 18 over to a rear half portion thereof, and a keyboard 21 is provided to be exposed in the opening 19. The substantially front half portion of the top cover 18 forms a palm rest 23. A touch pad 25 is provided in a central portion of the palm rest 23. Further, a plurality of exhaust vents, which will be described later, are provided in the side wall of the top cover 18 on a rear end side. The pair of hinges 15 are provided at the rear end portion of the top cover 18 and are disposed to be spaced from each other in the longitudinal direction of the first housing 12.

In this embodiment, the second housing 14 constitutes a display unit. The second housing 14 comprises a flat rectangular-shaped display housing 20 and a display panel 22 accommodated in the display housing 20. In the front wall of the display housing 20, a window 24 is formed for display. The window 24 has a size sufficient to cover most of the front wall, and the display surface of the display panel 22 is exposed to the outside of the display housing 20 through the window 24. The display panel 22 may be dedicated to normal display of images, text information, etc., or it may also serve as a touch panel that allows touch input, in addition to this.

The second housing 14 is supported by the pair of hinges 15 to be pivotable with respect to the first housing 12. With this structure, the second housing (display unit) 14 can be pivoted between a closed position where it is placed down to cover the top surface of the first housing 12 including the palm rest 23 and the keyboard 21, and an open position where it stands upright to expose the top surface of the first housing 12 and the display panel 22. Note that FIG. 1 shows the PC 10 when the second housing 14 is pivoted to the open position.

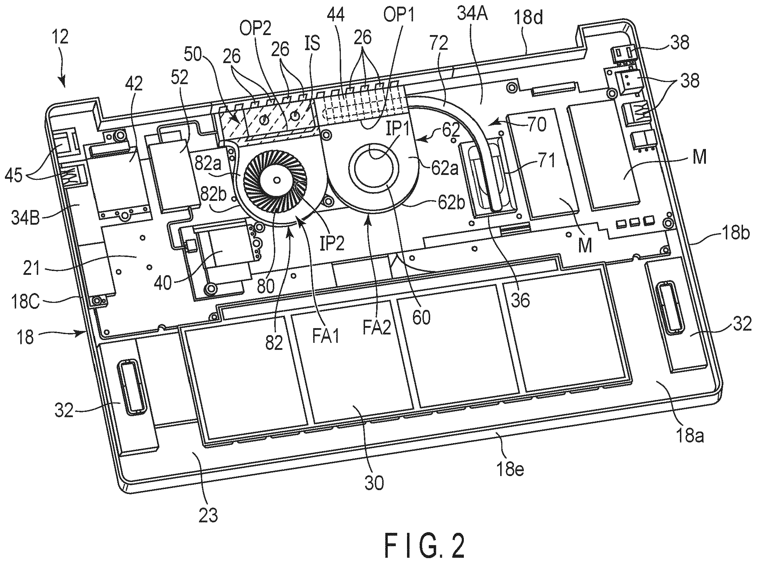

FIG. 2 is a perspective view showing an internal structure of the first housing 12 in a state where the first housing 12 is reversed from the state shown in FIG. 1 and the base 12 of the first housing 12 is removed.

As shown, the top cover 18 includes a rectangular-shaped top wall 18a, a pair of left and right side walls 18b and 18c, a rear side wall 18d and a front side wall 18e, which are formed, for example, of synthetic resin or metal to be integrated as one body. A plurality of vent ports 26 are provided in a longitudinal center portion of the rear side wall 18d. The vent ports 26 are spaced apart from one another in the longitudinal direction of the rear side wall 18d.

A battery 30 and a pair of speakers 32 are installed on the inner surface of the top wall 18a and are located on a rear surface side of the palm rest 23 described above. The keyboard 21 is located on the top wall 18a, between the battery 30 and the rear side wall 18d. Further, rectangular-shaped printed circuit boards 34A and 34B are disposed on the top wall 18a, overlaid on the keyboard 21 and located between the battery 30 and the rear side wall 18d. On the printed circuit board 34A, a plurality of electronic components is mounted, including a central processing unit (CPU) 36 and memories M, as heat-generating components, a storage device (for example, a solid state drive (SSD)) 40, a plurality of connectors 38 and the like. On the printed circuit board 34B, a plurality of electronic components including a communication module 42 and a plurality of connectors 45 are mounted.

Two cooling fans FA1 and FA2 are installed on the upper wall 18a of the top cover 18 and are located near the rear side wall 18d. The cooling fans FA1 and FA2 are fans that intake the internal air and heat of the first housing 12 and exhaust it outside the machine through the vent ports 26. The above-mentioned heat-generating components, namely, the CPU 36, the memory M, the battery 30, the SSD 40 and the communication module 42, are arranged around the cooling fans FA1 and FA2 to surround them. Thus, the cooling fans FA1 and FA2 contribute to the cooling of the electronic components by drawing the heat radiated from the electronic components and exhausting it to the outside of the PC 10.

In this embodiment, two cooling fans are provided, but the number is not limited to this. Depending on the cooling performance and the heat generation temperature in the machine, one or three or more cooling fans may be employed.

In this embodiment, heat-radiating fins 44 are provided between a discharge port of the cooling fan FA2 and the vent ports 26 of the first housing 12. An ion generator 50 is provided between a discharge port of the other cooling fan FA1 and the vent ports 26 of the first housing 12.

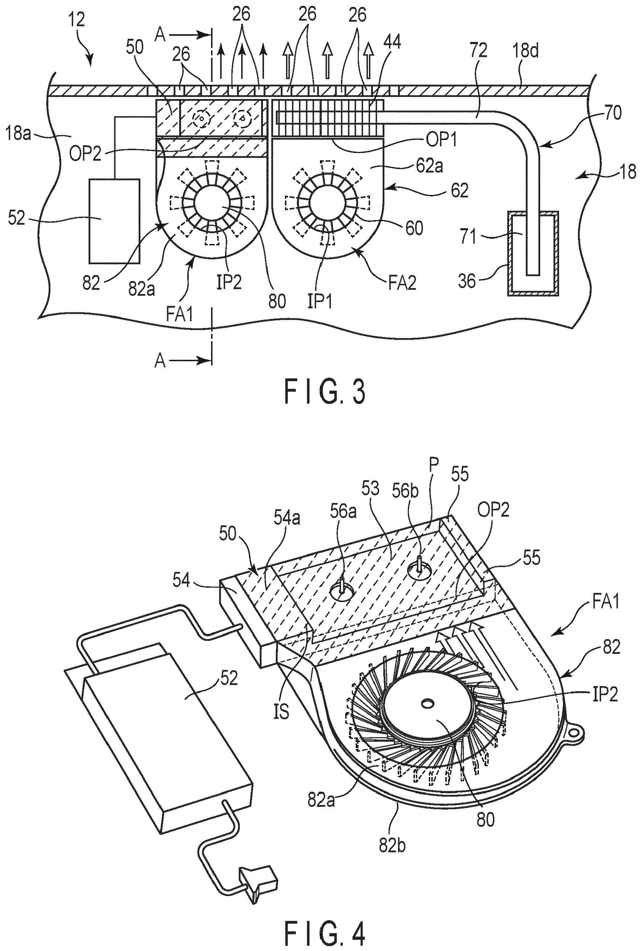

FIG. 3 is a plan view schematically showing the cooling fans, the ion generator and the cooling mechanism in the PC 10.

As shown in FIGS. 2 and 3, the cooling fan FA2 (corresponding to a second cooling fan) comprises a fan 60 and a fan case 62 enclosing the fan 60. The fan case 62 is formed of metal such as aluminum and includes an arc-shaped side frame, a top plate 62a and a lower plate 62b fixed to the side frame and opposing each other with a gap therebetween. Between the top plate 62a and the lower plate 62b, the fan 60 and a fan motor, not shown, are provided. The fan motor is supported by the lower plate 62b, for example. The fan case 62 comprises an intake port IP1 formed in the top plate 62a and a discharge port OP1 formed in the side frame.

The cooling fan FA2 is arranged so that the lower plate 62b overlaps the top wall 18a of the top cover 18 and the rotation axis of the fan 60 is substantially orthogonal to the top wall 18a. The discharge port OP1 of the fan case 62 opposes the rear side wall 18d of the top cover 18 substantially parallel to each other with a gap therebetween, and opposes the vent ports 26. The intake port IP1 of the fan case 62 is open to the interior of the first housing 12.

The PC 10 further comprises a cooling unit 70 for actively cooling the CPU 36, which generates a relatively great amount of heat. The cooling unit 70 is provided inside the first housing 12. The cooling unit 70 includes a heat-radiating plate 71 formed of a metal with high heat-radiating property such as aluminum, heat-radiating fins 44 including a great number of fins formed of aluminum or the like and arranged into a substantially rectangular shape as a whole, and a heat pipe 72 thermally connecting the heat-radiating plate 71 and the heat-radiating fins 44.

The heat-radiating plate 71 is disposed in contact with the surface of the CPU 36 via a heat-transfer sheet (not shown). The heat-radiating fins 44 are disposed between the discharge port OP1 of the cooling fan FA2 and the vent ports 26 of the first housing 12. The heat-radiating fins 44 are each provided in a direction approximately perpendicular to the rear side wall 18d and are arranged parallel with each other with a predetermined interval therebetween in the longitudinal direction of the rear side wall 18d. One end of the heat pipe 72 is connected to the plurality of fins.

According to the cooling unit 70, the heat generated by the CPU 36 is transferred to the heat pipes 72 via the heat transfer sheet and the heat-radiating plate 71, and further to the heat-radiating fins 44 via the heat pipes 72. The transmitted heat is then radiated from the heat-radiating fins 44. At that time, the cooling fan FA2 is operated, and thus the internal air and heat in the first housing 12 are suctioned into the fan case 62 from the intake port IP1, compressed and sent from the discharge port OP1 to the vent ports 26 via the heat-radiating fins 44, and exhausted from the vent ports 26 to the outside of the machine together with the heat radiated from the heat-radiating fins 44. Thus, the CPU 36 and the inside of the first housing 12 are cooled.

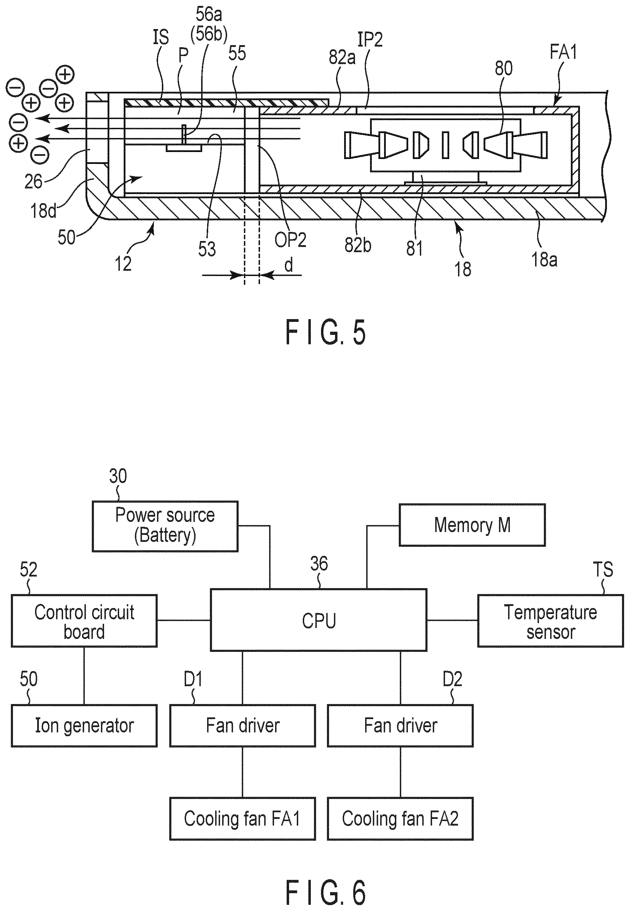

As shown in FIGS. 2 and 3, the cooling fan FA1 comprises a fan 80 and a fan case 82 enclosing the fan 80. The fan case 82 is made of metal such as aluminum, and includes an arc-shaped side frame, a top plate 82a and a lower plate 82b fixed to the side frame and opposing each other with a gap therebetween. The fan 80 and a fan motor 81 are provided between the top plate 82a and the lower plate 82b. The fan motor 81 is supported by the lower plate 82b, for example (See FIG. 5). The fan case 82 includes an intake port IP2 formed in the top plate 82a and a discharge port OP2 formed in the side frame.

The cooling fan FA1 is arranged so that the lower plate 82b overlaps the top wall 18a of the top cover 18 and the rotation axis of the fan 80 is substantially orthogonal to the top wall 18a. The discharge port OP2 of the fan case 82 opposes parallel to the rear side wall 18d of the top cover 18 with a gap therebetween, and also opposes a plurality of vent ports 26. The intake port IP2 of the fan case 82 is open to the interior of the first housing 12. In this embodiment, the cooling fan FA1 is arranged along the cooling fan FA2 in the longitudinal direction of the first housing 12.

The ion generator 50 is disposed between the discharge port OP2 of the cooling fan FA1 and the vent ports 26 of the first housing 12. The ion generator 50 is formed into a rectangular box shape of dimensions approximately equal to those of the heat-radiating fins 44. In place of the heat-radiating fins, the ion generator 50 is disposed at the location of the heat-radiating fins, that is, between the discharge port OP2 of the cooling fan FA1 and the rear side wall 18d. A control circuit board 52, which controls the operation of the ion generator 50, is installed on the top wall 18a and located between the cooling fan FA1 and the communication module 42. The control circuit board 52 is connected to the ion generator 50 via wiring lines.

FIG. 4 is a perspective view showing the cooling fan FA1 and the ion generator 50, and FIG. 5 is a cross-sectional view of the cooling fan and the ion generator taken along line A-A in FIG. 3.

As shown in FIG. 4, the ion generator 50 has a flat rectangular box-shaped housing 54 formed of an insulating material such as synthetic resin. A rectangular-shaped recess 53 is formed in a longitudinal region of one of the main surfaces 54a of the housing 54. The recess 53 is open to the main surface 54a and a pair of side surfaces of the housing 54. A side wall 55 is provided to stand on one longitudinal end side of the recess 53. The height of the side wall 55 coincides the height of the main surface 54a.

The ion generator 50 includes two discharge electrodes 56a and 56b provided in the housing 54, which are electrically connected to the control circuit board 52 via wiring lines (not shown). The discharge electrodes 56a and 56b protrude from the bottom of the recess 53 into the recess 53 and are exposed in the recess 53. The discharge electrodes 56a and 56b are arranged at intervals in the longitudinal direction of the housing 54. By applying positive (+) and negative (-) voltages to the discharge electrodes 56a and 56b from the control circuit board 52, plasma discharge occurs to generate positive ions (H+) of hydrogen and negative ions (O2-) of oxygen from water and oxygen in the air.

As shown in FIGS. 4 and 5, the ion generator 50 opposes the fan case 82 with a predetermined gap d therebetween. The gap d is set appropriately to secure a spatial distance between the discharge electrodes 56a and 56b of the ion generator 50 and the fan case 82 made of metal. With the gap d thus set, the adverse effect of the fan case 82 on the plasma discharge and the noise produced by the fan can be reduced.

An insulating sheet IS is attached over the main surface 54a of the ion generator 50 and the outer surface of the top plate 82a of the cooling fan FA1. The insulating sheet IS extends to the extending end of the side wall 55 and is also attached to the extending end. The insulating sheet IS covers the recess 53 of the housing 54 and opposes the bottom surface of the recess 53 with a gap therebetween. Further, the insulating sheet IS constitutes a cover member and covers the gap d between the cooling fan FA1 and the ion generator 50. That is, the insulating sheet IS blocks an upper opening of the gap d. The insulating sheet IS and the recess 53 define a tunnel-shaped passage channel P connecting the discharge port OP2 of the cooling fan FA1 and the respective vent port 26 of the first housing 12. The pair of discharge electrodes 56a and 56b are exposed in the passage channel P. As described above, to avoid the effect of metal on the ion generation, the insulating sheet IS, instead of a metal cover, is attached to the main surface 54a of the ion generator 50 and the fan case 82, thus making it possible to form a tunnel-shaped passage channel P with secured spatial and creepage distances.

When the cooling fan FA1 is operated, the internal air and heat inside the first housing 12 are suctioned into the fan case 82 from the intake port IP2, compressed by the fan 80, sent from the discharge port OP2 to the vent ports 26 via the passage channel P of the ion generator 50, and exhausted to the outside of the machine from the vent ports 26. At the same time, the positive ions of hydrogen (H+) and negative ions of oxygen (O2-) generated by the plasma discharge of the ion generator 50 are released from the vent ports 26 to the outside of the machine by the compressed air passing through the passage channel P of the ion generator 50, and become cluster ions with water molecules attached therearound. The released cluster ions decompose the proteins on the surfaces of floating bacteria and viruses floating in the outside air, to inhibit their actions, thereby purifying the air.

The control operation of the cooling fans FA1 and FA2 in PC 10 will now be described.

FIG. 6 is a block diagram schematically showing the control system of the PC 10. As shown, the CPU 36, which functions as the controller, drives the cooling fans FA2 according to the temperature inside the first housing 12, in particular, the temperature detected by a temperature sensor TS which detects the temperature of the CPU 36. For example, when the detected temperature rises to a predetermined temperature or higher, the CPU 36 drives the cooling fan FA2 via the fan driver D2 to cool the CPU 36 and the interior of the first housing 12. The CPU 36 continuously drives the cooling fan FA1 until the detected temperature falls to a set temperature or lower. In the state below the set temperature, the CPU 36 may stop the cooling fan FA2 or may drive the cooling fan FA2 constantly at a low speed.

Meanwhile, the CPU 36 controls the operation of the other cooling fan FA1 according to the operation status of the ion generator 50. When an operating software (OS) stored in the memory M sets the ion generator 50 to operate at regular intervals, or when the ion generator 50 is set to be on by the operator's instruction, the CPU 36 drives the cooling fan FA1 via the fan driver D1 when the ion generator 50 operates, to send the compressed internal air to the ion generator 50. Thus, the positive ions of hydrogen (H+) and negative ions of oxygen (O2-) generated by the plasma discharge of the ion generator 50 are diffused out of the machine through the vent ports 26.

In the PC 10 of this embodiment, the two cooling fans FA1 and FA2 and the cooling unit 70 are used to control the temperature of the CPU 36 and to cool the inside of the first housing 12. When the load of the CPU 36 is light, the amount of heat generated is low, so that the temperature of the CPU 36 can be controlled even with one cooling set. Thus, even if a plurality of CPU cooling mechanisms are provided, a single cooling set may be sufficient depending on the conditions. According to this embodiment, in a notebook personal computer, a small-sized ion generator of substantially the same dimensions as those of the heat-radiating fins is exclusively mounted in the space of one of the cooling mechanisms. With this structure, the housing of the PC 10 can be applied without increasing its size, and the influence on the mounting of other components can be reduced to avoid the loss of the other functions of the PC 10.

The ion generator 50 is formed to have substantially the same outer size as that of the heat-radiating fins 44, and is placed on a discharge port side of the cooling fan FA1 as in the case of the heat-radiating fins. The air compressed by the cooling fan FA1 is sent to the vent ports while passing through the discharge electrodes 56 and 56b of the ion generator 50. At this time, to avoid the effect of metal on the ion generation, the insulating sheet IS, in place of the metal cover of the cooling fan, is attached to the main surface of the ion generator 50 and the fan case, thereby forming the tunnel-shaped passage channel P with secured spatial and creepage distances. With this structure, the air compressed by the cooling fan FA1 can be efficiently passed through the passage channel P in which the discharge electrodes are arranged, and the generated ions can be efficiently released to the outside of the machine.

As described above, according to this embodiment, a cooling fan for cooling PC is used to enable ion diffusion and there is no need to install a fan dedicated to ion diffusion, and thus it is possible to provide an electronic device that can both save space and achieve an ion generation function at the same time.

In the above-described embodiment, in place of providing an insulating sheet IS, it may be replaced by an insulating plate member integrated with the housing 54 of the ion generator 50. In other words, it may be used as a housing 54 comprising a tunnel-shaped passage channel with a closed upper surface.

Next, electronic devices of other embodiments, such as PCs, will be described. In the other embodiments described below, the same structural parts as those of the first embodiment described above are denoted by the same reference signs as those of the first embodiment, and the detailed description thereof may be omitted or simplified.

### (Second Embodiment)

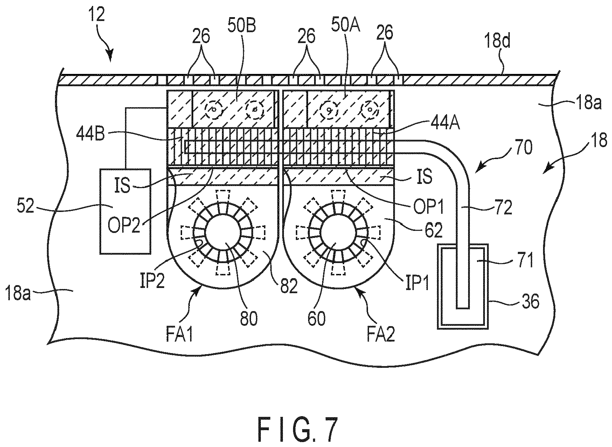

FIG. 7 is a plan view schematically showing a cooling fan, an ion generator and a cooling mechanism of a personal computer according to a second embodiment.

As shown in FIG. 7, according to the second embodiment, heat-radiating fins 44A are provided adjacent and opposite to the discharge port OP1 of the cooling fan FA2, and further, an ion generator 50A is installed between the heat-radiating fins 44A and vent ports 26 of a rear side wall 18d. Heat-radiating fins 44B are provided adjacent and opposite to the discharge port OP2 of the other cooling fan FA1, and further, an ion generator 50B is installed between the heat-radiating fins 44B and the vent ports 26 of the rear side wall 18d.

The heat-radiating fins 44A and 44B are mechanically and thermally connected to one end portion of the heat pipe 72. The ion generators 50A and 50B are each formed to have dimensions and a structure similar to those of the ion generators 50 in the first embodiment described above. The ion generators 50A and 50B are connected to the control circuit board 52 via wiring lines, and thus their operations are controlled by the control circuit board 52.

As described above, a cooling unit 70 including heat-radiating fins are provided at the cooling fans FA2 and FA1, respectively, and the ion generators 50A and 50B are disposed, respectively, between the discharge ports of both the cooling fans FA2 and FA1 and the vent ports 26 of the rear side wall 18d. In the case of the above-discussed configuration, the CPU 36 controls the operation of the cooling fan FA2 according to the internal temperatures of the CPU 36 and the first housing 12 and according to the operating state of the ion generator 50A. Similarly, the CPU 36 controls the operation of the cooling fan FA1 according to the internal temperatures of the CPU 36 and the first housing 12 and according to the operating state of the ion generator 50B.

According to the second embodiment configured as described above, ions can be diffused using the cooling fans FA1 and FA2 for cooling, thereby making it possible to achieve both space saving and ion generation functions at the same time as in the case of the first embodiment described above. Further, two sets of heat-radiating fins 44A and 44B and two sets of ion generators 50 are installed, and thus it is possible to increase the amount of ion diffusion while maintaining the cooling performance of the electronic device.

### (Third Embodiment)

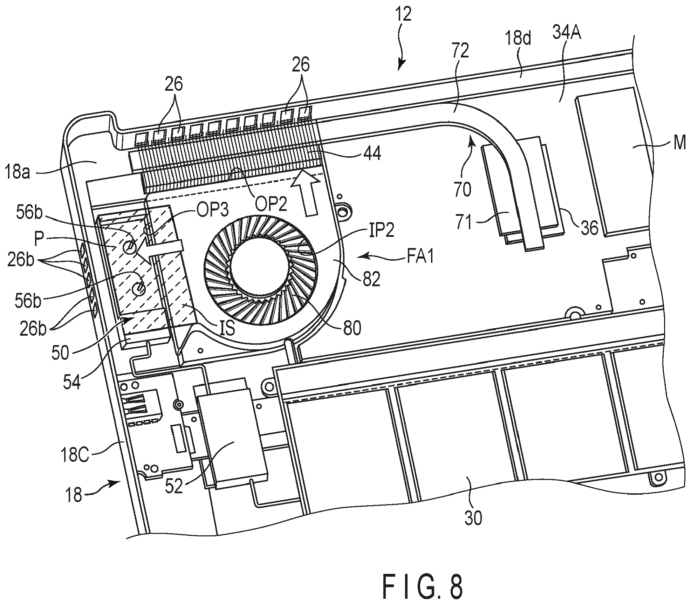

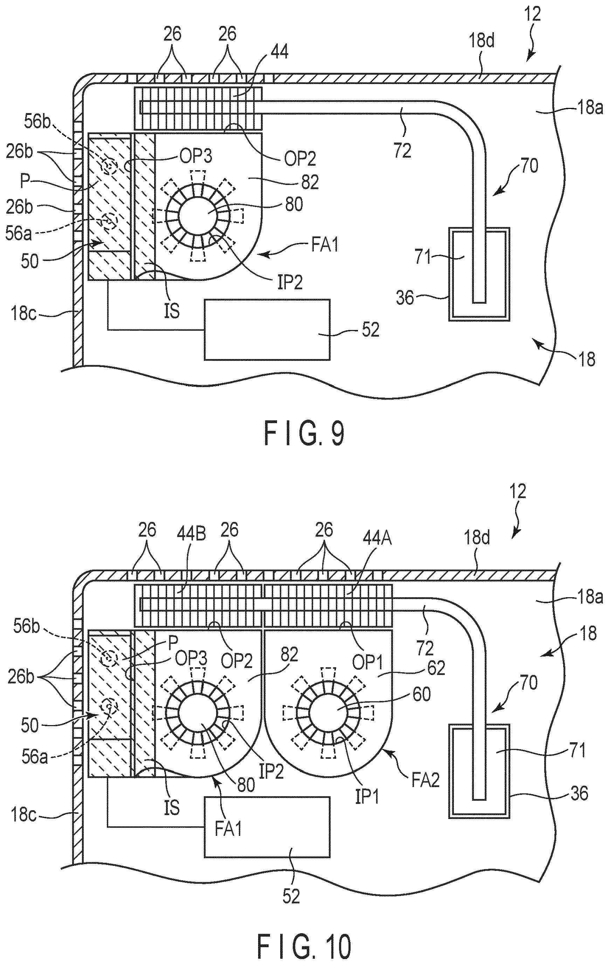

FIG. 8 is a perspective view showing a cooling fan, an ion generator and a cooling mechanism in a personal computer according to a third embodiment, and FIG. 9 is a plan view schematically showing the cooling fan, the ion generator and the cooling mechanism of the personal computer according to the third embodiment. As shown, according to the third embodiment, the PC 10 comprises a single cooling fan FA1 provided in the first housing 12. The cooling fan FA1 is provided in a corner portion of the first housing 12. The rear side wall 18d of the first housing 12 includes a plurality of vent ports 26 provided near the corner portion. The vent ports 26 are arranged to be spaced away from each other in the longitudinal direction of the rear side wall 18d. Further, the side wall 18c of the first housing 12 that intersects the rear side wall 18d to form the corner portion includes a plurality of vent ports 26b provided in the vicinity of the corner portion. The vent ports 26b are arranged to be spaced apart from each other in the longitudinal direction of the side wall 18c.

The cooling fan FA1 comprises a fan 80 and a fan case 82 enclosing the fan 80. The fan case 82 is formed of a metal such as aluminum. The fan case 82 includes an air intake port IP2 formed in the top plate 82a and two discharge ports OP2 and OP3 formed in the side frame.

The cooling fan FA1 is installed on the top wall 18a so that the axis of rotation of the fan 80 is substantially orthogonal the top wall 18a. The discharge port OP2 of the fan case 82 opposes substantially parallel to the rear side wall 18d with a gap therebetween and also opposes a plurality of vent ports 26. The discharge port OP3 of the fan case 82 opposes substantially parallel to the side wall 18c with a gap therebetween and also opposes a plurality of vent ports 26b. The intake port IP2 of the fan case 82 is open to the interior of the first housing 12.

Heat-radiating fins 44 are installed between the discharge port OP2 of the cooling fan FA1 and the rear side wall 18d so as to oppose the vent ports 26. The heat-radiating fins 44 are thermally connected to a heat-radiating plate 71 via a heat pipe 72. The heat-radiating plate 71 is overlaid on the CPU 36. An ion generator 50 is installed between the discharge port OP3 of the cooling fan FA1 and the side wall 18c, so as to oppose the vent ports 26b. The ion generator 50 is connected to a control circuit board 52 via wiring lines, and its operation is controlled by the control circuit board 52.

The ion generator 50 is configured to be similar to the ion generator in the embodiments described above. That is, the ion generator 50 comprises a rectangular box-shaped housing 54 and two discharge electrodes 56a and 56b provided in the housing 54. The electrodes 56a and 56b are electrically connected to the control circuit board 52 via wiring lines (not shown). The discharge electrodes 56a and 56b are exposed in the recess of the housing 54. An insulating sheet IS is attached over the main surface of the ion generator 50 and the outer surface of the cooling fan FA1. The insulating sheet IS covers the recess of the housing 54 and also covers the gap between the cooling fan FA1 and the ion generator 50. The insulating sheet IS and the recess define a tunnel-shaped passage channel P which connects the discharge port OP3 of the cooling fan FA1 and the vent ports 26b of the first housing 12. The pair of discharge electrodes 56a and 56b are exposed in the passage channel P.

In the above configuration, the CPU 36 controls the operation of the cooling fan FA1 according to the internal temperatures of the CPU 36 and the first housing 12 and according to the operating state of the ion generator 50.

According to the third embodiment configured as described above, ions can be diffused using a cooling fan for cooling, thereby making it possible to achieve both space saving and ion generation functions at the same time as in the case of the first embodiment described above. With two discharge ports OP2 and OP3 provided on the cooling fan FA1, the heat-radiating fins 44 are provided to oppose one discharge port OP2 and the ion generator 50 is provided to oppose the other discharge port OP3. With this structure, even in the case of a single cooling fan provided, the cooling function and the ion generating function can be achieved at the same time.

In the third embodiment, it is also possible to install the ion generator 50 on a rear side wall 18d side and the heat-radiating fins 44 on a side wall 18c side.

### (Fourth Embodiment)

FIG. 10 is a plan view schematically showing cooling fans, an ion generator and a cooling mechanism of a personal computer according to a fourth embodiment.

As shown, according to the fourth embodiment, a further cooling fan FA2 is provided for cooling for the PC 10 of the third embodiment described above. The cooling fan FA2 is arranged along the cooling fan FA1. The fan case 62 of the cooling fan FA2 includes a discharge port OP1 and an intake port IP1. The cooling fan FA2 is arranged so that the discharge port OP1 opposes parallel to the rear side wall 18d with a gap therebetween.

Heat-radiating fins 44A are installed between the discharge port OP1 of the cooling fan FA2 and the rear side wall 18d so as to oppose the vent ports 26. The heat-radiating fins 44A are thermally connected to the heat-radiating plate 71 via a heat pipe 72. In the fourth embodiment, the other configuration of the PC 10 is identical to that of the PC of the third embodiment described above.

In the above configuration, the CPU 36 controls the operation of the cooling fan FA2 according to the internal temperatures of the first housing 12 and the CPU 36. Further, the CPU 36 controls the operation of the cooling fan FA1 according to the internal temperatures of the first housing 12 and the CPU 36 and according to the operation state of the ion generator 50.

According to the fourth embodiment configured as described above, ions can be diffused using a cooling fan for cooling, thereby making it possible to achieve both space saving and ion generation functions at the same time as in the case of the third embodiment described above. With two discharge ports OP2 and OP3 provided on the cooling fan FA1, the heat-radiating fins 44 are provided to oppose one discharge port OP2 and the ion generator 50 is provided to oppose the other discharge port OP3. With this structure, the cooling fan FA1 can achieve the cooling function and the ion generating function at the same time. Further, according to the fourth embodiment, the cooling performance of the CPU can be improved by adding a cooling fan FA2.

While certain embodiments have been described, these embodiments have been presented by way of example only, and are not intended to limit the scope of the inventions. Indeed, the novel embodiments described herein may be embodied in a variety of other forms; furthermore, various omissions, substitutions and changes in the form of the embodiments described herein may be made without departing from the inventions. The accompanying claims and their equivalents are intended to cover such forms or modifications as would fall within the scope of the inventions.

For example, the electronic devices are not limited to personal computers, but the invention can be applied to various other electronic devices. The heat-generating components located inside the housing of the electronic devices are not limited to the CPU, but also include other electronic components such as memories, batteries, etc.

The shape and material of the cooling fans and heat-radiating fins are not limited to those of the embodiments provided above, but can be changed to other shapes and materials as appropriate.

In the above-described embodiments of the invention, the CPU 36 controls the operation of the other cooling fan FA1 according to the operation status of the ion generator 50, but the configuration is not limited to this. It is possible that the operation of the cooling fan FA1 and/or the operation of the ion generator 50 can be controlled by a power supply control circuit which can communicate with the CPU 36. Thus, ion generation and ion diffusion by the ion generator 50 can be carried out regardless of the drive status of the PC itself. The power control circuit may be connected to a network circuit (not shown) to be communicatable, and the operations of the cooling fan FA1 and the ion generator 50 may be controlled based on the control signals from the network circuit. Thus, it is possible to control the operation of the cooling fan FA1 and the ion generator 50 by remote control.

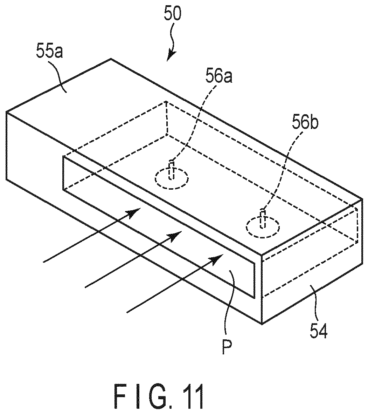

As described above, the ion generator may be configured to include a component part corresponding to the insulating sheet as a part integrated thereto. For example, as shown in FIG. 11, the housing 54 of the ion generator 50 may be formed into a rectangular box shape comprising a tunnel-shaped passage channel P extending to penetrate the pair of side walls opposing each other, with use of an insulating material, for example, synthetic resin, in place of the insulating sheet.

## Claims

1. An electronic device **characterized by** comprising:
a housing (12) including a wall portion with a vent port (26);
a plurality of electronic components including a heat-generating component (36), disposed within the housing;
a cooling fan (FA1) including a discharge port (OP2) opposing the vent port and an intake port (IP2) opening into the housing, and arranged inside the housing to suction internal air of the housing and discharge the air from the discharge port toward the vent port; and
an ion generator (50) disposed inside the housing between the discharge port of the cooling fan and the vent port, and including a passage channel (P) communicating with the discharge port and the vent port and discharge electrodes (56a, 56b) exposed in the passage channel.

2. The electronic device of claim 1, **characterized in that**
the cooling fan (FA1) comprises a fan case (82) including the intake port (IP2) and the discharge port (OP2), and a fan disposed in the fan case; and
the ion generator (50) is disposed to oppose the discharge port and to be spaced away from the fan case with a gap, and further comprises a cover member covering the gap.

3. The electronic device of claim 2, **characterized in that**
the ion generator (50) comprises a box-shaped housing (54) with a recess (53) and the discharge electrodes (56a, 56b) provided in the housing (54) and exposed in the recess, and
the cover member includes an insulating sheet (IS) attached over the housing of the ion generator and the fan case (82) of the cooling fan (FA1), and
the insulating sheet covers the recess and defines the passage channel (P) in a form of a tunnel and extends over the gap.

4. The electronic device of claim 2, **characterized in that**
the ion generator (50) includes a housing (54) including a tunnel-shaped passage channel (P), and the discharge electrodes (56a, 56b) provided in the housing and exposed to the passage channel.

5. The electronic device of claim 1, **characterized in that**
the cooling fan (FA1) includes a second discharge port (OP3) opening in a direction different from that of the discharge port (OP2);
the housing (12) includes a second wall portion provided with a plurality of second vent ports (26b) opposing the second discharge port; and
**characterized by** further comprising heat-radiating fins (44) provided between the second discharge port and the second vent ports.

6. The electronic device of claim 5, **characterized by** further comprising a heat-radiating plate (71) overlaid on the heat-generating component (36), and a heat pipe (72) including one end connected to the heat-radiating fins (44) and the other end connected to the heat-radiating plate.

7. The electronic device of claim 1, **characterized by** further comprising heat-radiating fins (44B) provided between the discharge port (OP2) of the cooling fan (FA1) and the ion generator (50); a heat-radiating plate (71) overlaid on the heat-generating component (26); and a heat pipe (72) including one end connected to the heat-radiating fins and the other end connected to the heat-radiating plate.

8. The electronic device of any one of claims 5 to 7, **characterized by** further comprising a controller (36) which controls operation of the cooling fan according to a temperature of the heat-generating component (36) or a temperature inside the housing (12) and according to an operating state of the ion generator (50).

9. The electronic device of claim 1, **characterized by** further comprising a second cooling fan (FA2) including a discharge port (OP1) opposing the vent port (26) and an intake port (IP1) opening into the housing (12) and disposed in the housing in line with the cooling fan (FA1), heat-radiating fins (44A) provided between the discharge port of the second cooling fan (FA2) and the vent port, a heat-radiating plate (71) overlaid on the heat-generating component (36), and a heat pipe (72) including one end connected to the heat-radiating fins and the other end connected to the heat-radiating plate.

10. The electronic device of claim 9, **characterized by** further comprising a controller (36) which controls operation of the cooling fan (FA1) according to an operating state of the ion generator (50), and controls operation of the second cooling fan (FA2) according to a temperature of the heat-generating component (36) or a temperature inside the housing.

11. The electronic device of claim 1, **characterized in that**
the electronic components include a central processing unit (36), a battery (30), a memory (M), a storage device (40), and a communication module (42), disposed around the cooling fan (FA1).
